(19) Europäisches Patentamt
European Patent Office
Office européen des brevets

(11) EP 1 285 962 B1

(12) EUROPÄISCHE PATENTSCHRIFT

(45) Veröffentlichungstag und Bekanntmachung des Hinweises auf die Patenterteilung:
**13.02.2008 Patentblatt 2008/07**

(51) Int Cl.:
*C12N 9/02* (2006.01) *C12P 13/04* (2006.01)
*C12P 7/04* (2006.01)

(21) Anmeldenummer: **02017569.1**

(22) Anmeldetag: **07.08.2002**

# (54) NADH-Oxidase aus Lactobacillus

NADH oxidase from Lactobacillus

NADH oxidase de Lactobacillus

(84) Benannte Vertragsstaaten:
**AT BE BG CH CY CZ DE DK EE ES FI FR GB GR IE IT LI LU MC NL PT SE SK TR**

(30) Priorität: **16.08.2001 DE 10140088**

(43) Veröffentlichungstag der Anmeldung:
**26.02.2003 Patentblatt 2003/09**

(73) Patentinhaber: **Evonik Degussa GmbH
40474 Düsseldorf (DE)**

(72) Erfinder:
- **Hummel, Werner, Dr.
52445 Titz (DE)**
- **Bommarius, Bettina, Dr.
Altanta, GA 30327 (US)**

(56) Entgegenhaltungen:
**WO-A-01/23582**

- **DATABASE EMBL [Online] 15. Dezember 2000 (2000-12-15) KNORR ET AL: "Genomic region of the gene encoding for a putative NADH oxidase from Lactobacillus sanfranciscensis" Database accession no. AB035801 XP002219891**
- **YI XIANWEN ET AL: "Properties of NADH oxidase from Lactobacillus delbrueckii ssp bulgaricus." JOURNAL OF THE SCIENCE OF FOOD AND AGRICULTURE, Bd. 78, Nr. 4, Dezember 1998 (1998-12), Seiten 527-534, XP002219890**
- **DATABASE EMBL [Online] 3. Februar 1993 (1993-02-03) ROSS & CLAIBORNE: "Molecular cloning and analysis of the gene encoding the NADH oxidase from Streptococcus faecalis 10C1" Database accession no. X68847 XP002219892 & ROSS & CLAIBORNE: "Molecular cloning and analysis of the gene encoding the NADH oxidase from Streptococcus faecalis reductase" J. MOL. BIOL., Bd. 227, 1992, Seite 658-671**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung ist auf eine NADH-Oxidase aus Lactobacillus gerichtet, sowie die dieses Enzym codierenden Nukleinsäuren und Vehikel, aufweisend diese Nukleinsäuren.

Insbesondere richtet sich die Erfindung auf eine Oxidase aus *Lactobacillus brevis* (DSM 20054).

Dehydrogenasen, klassifiziert unter E.C. 1.1, sind Enzyme, die reversibel die Oxidation oder Reduktion bestimmter Verbindungen, wie z.B. Alkohole und Ketone, katalysieren. Einige der bekannten Dehydrogenasen sind cofaktorabhängig, sprich sie benötigen weitere Moleküle zum Ausgleich der Elektronentransferprozesse. Als solche Cofaktoren sind z.B. NADH und NADPH bekannt.

Gemäß nachstehendem Schema wird bei der Oxidation von z.B. Alkoholen zu Ketonen NADH gebildet. Um nicht stöchiometrische Mengen an teurem NAD+ einsetzen zu müssen, kann man durch ein zweites Enzymsystem die Bildung von NAD+ initiieren (Enzyme Catalysis in Organic Synthesis, Ed.: K. Drauz, H. Waldmann, VCH, 1. Auflage, S. 721; Schema 1).

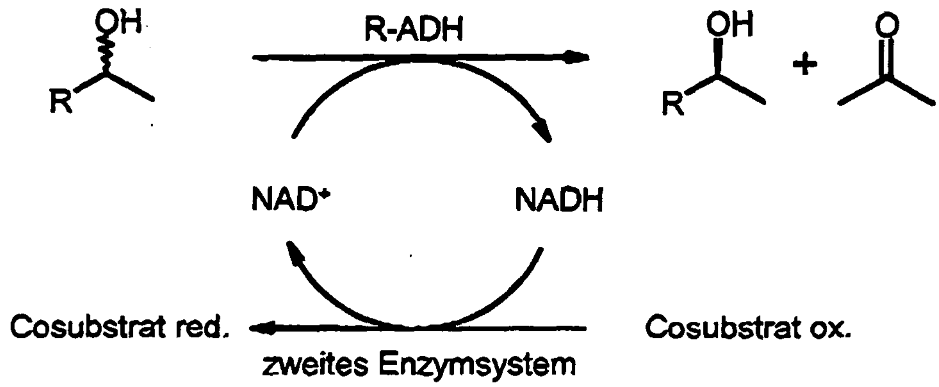


Literaturbekannte Systeme nutzen für die Regenerierung von NAD+ beispielsweise Lactat-Dehydrogenase und Pyruvat unter Lactat-Bildung oder Glutamat-Dehydrogenase mit Ketoglutarat und Ammonium unter Bildung von Glutamat. Nachteilig ist dabei, daß Hilfssubstrate wie Pyruvat oder Ketoglutarat eingesetzt werden müssen und Produkte wie Lactat oder Glutamat gebildet werden, die bei präparativer Nutzung vom eigentlich gebildeten Produkt entfernt werden müssen. Zudem handelt es sich hierbei um Gleichgewichtsreaktionen, die nur schwierig reaktionstechnisch einen vollständigen Ablauf der Reaktion ermöglichen.

Alternativ kann ein solches zweites Enzymsystem eine NADH-Oxidase sein, welche als oxidiertes Cosubstrat Luftsauerstoff akzeptiert und unter Bildung von NAD+ gleichzeitig Wasser oder Wasserstoffperoxid generiert. NADH-Oxidasen bieten in dieser Hinsicht den Vorteil, daß die von ihnen katalysierte Reaktion irreversibel ist, $O_2$ als Regenerierungssubstrat verwendet wird und $H_2O$ oder $H_2O_2$ als Produkte gebildet werden. Enzyme beider Gruppen ($H_2O$- und $H_2O_2$-bildende) sind in der biochemischen Literatur im Prinzip bekannt (z.B. für $H_2O$-bildende Enzyme s. Lopez de Felipe, F. et al., J. Bacteriol. Vol. 180 (1998), 3804-08; für $H_2O_2$-bildende Enzyme Nishiyama, Y. et al. J. Bacteriol. Vol. 183 (2001), 2431-2438 und jeweils zitierte Literatur). Für die Verwendung als Regenerierungsenzym sind H2O2-bildende Enzyme weniger gut geeignet, da Peroxid als enzymschädigend bekannt ist und möglichst *in situ* abgebaut werden sollte, was weitere eher nachteilige jedoch nicht unmöglich Prozeßschritte erforderlich macht.

Biochemisch charakterisiert sind die NADH-Oxidasen aus *Enterococcus (bzw. Streptococcus) faecalis* (Schmidt, H.L. et al., Eur. J. Biochem. 156 (1986), 149-55), *Leuconostoc mesenteroides* (Koike, K. J. et al., Biochem. (Tokyo) 97 (1985), 1279-88), *Streptococcus mutans* (Higuchi, M. et al., Biosci. Biotechnol. Biochem. 58 (1994), 1603-07), *Mycoplasma capricolum* (Klomkes, M., Altdorf, R., Ohlenbusch, H. D., Biol. Chem. Hoppe Seyler 366 (1985), 963-9), *Sulfolobus solfataricus* (Arcari, P. et al., J. Biol. Chem. 275 (2000), 895-900), Thermus thermophilus (Erdmann, H. et al., J. Mol. Biol. 230 (1993), 1086-8) und aus *Thermus aquaticus* (Cocco, D. et al., Eur. J. Biochem. 174 (1988), 267-71).

Aufgabe der vorliegenden Erfindung war es eine NADH-Oxidase mit hoher Aktivität zur Verfügung zu stellen, welche sich im gekoppelten Einsatz mit Dehydrogenasen für die NAD+-Regenerierung eignet. Insbesondere sollte die NADH-Oxidase leicht herstellbar sein und in ausreichender Menge für den vorteilhaften Einsatz im technischen Maßstab zur Verfügung stehen.

Die Aufgabe wird anspruchsgemäß gelöst. Anspruch 1 bezieht sich auf eine NADH-Oxidase (NOX) aus der Gattung Lactobacillus. Anspruch 2 richten sich auf bevorzugte Enzyme. Anspruch 3 stellt die diese Enzyme codierenden Nukleinsäuren unter Schutz. Anspruch 4 wiederum betrifft Vehikel, welche die erfindungsgemäßen Nukleinsäuren aufweisen. Anspruch 5 schützt bevorzugte Primer. Anspruch 6 und 17 richten sich auf bestimmte Verwendungen der erfindungsgemäßen NADH-Oxidasen (NOX) bzw. erfindungsgemäßen Nukleinsäuren. Anspruch 8 ist auf einen erfindungsgemäßen Mikroorganismus gerichtet. Anspruch 9 umfasst ein Verfahren zur Herstellung der NADH-Oxidasen aus Lactobacillus.

Dadurch, daß man eine NADH-Oxidase (NOX) aus Lactobacillus-Arten zur Verfügung stellt, besitzt man die Möglichkeit, die gestellte Aufgabe vorteilhaft zu lösen.
Ganz besonders bevorzugt ist dabei der Einsatz einer NADH-Oxidase (NOX) aus *Lactobacillus brevis*, [DSM 20054 (Seq. 2)].

Gemäß eingangs gezeigtem Schema ist die NADH-Oxidase in der Lage, das bei der Reaktion entstehende NADH in NAD+ umzuwandeln. Aufgrund der Irreversibilität der Reaktion $O_2$ zu $H_2O$ erfolgt durch den Gleichgewichtsdruck eine vollständige Umsetzung des eingesetzten racemischen Alkohols zu Keton unter Zurückbleiben einer hoch enantiomerenangereicherten nicht umgesetzten optischen Antipode des eingesetzten Alkohols. Beispiele für die Anwendung der NADH-Oxidase (NOX) sind u.a. die Herstellung von R-Alkoholen in Kopplung mit einer S-Alkohol-Dehydrogenase, von S-Alkoholen in Kopplung mit einer R-Alkohol-Dehydrogenase oder von D-Aminosäuren in Kopplung mit einer L-Aminosäure-Dehydrogenase. Ebenso ist in diesem Sinne die Kopplung mit Hydroxysäure-Dehydrogenasen und allen anderen NAD-abhängigen Dehydrogenasen möglich. Als weitere wichtige Produkte kommen auch die Oxidationsprodukte der Dehydrogenase-Reaktion, also beispielsweise Ketone, Ketosäuren oder Aldehyde in Betracht.

In einer nächsten Ausgestaltung beschäftigt sich die Erfindung mit Nukleinsäuren codierend für eine erfindungsgemäße NADH-Oxidase.

Trotz der relativ leichten Kultivierbarkeit der Lactobacillus-Stämme und einfachen Zugänglichkeit des Enzyms über chromatographische Methoden gelangt man durch die Angabe der Nukleinsäuren codierend für eine erfindungsgemäße NADH-Oxidase (NOX) in weiterhin bevorzugter Art und Weise zu Substanzen, welche es erlauben, die für einen enzymatisch-technischen Prozeß zur Erzeugung von enantiomerenangereicherten Verbindungen notwendigen Enzyme in ausreichender Menge über rekombinante Techniken sicherzustellen. Es ist mit den Nukleinsäuren möglich, die Enzyme aus schnell wachsenden Wirtsorganismen in hohen Ausbeuten zu gewinnen. Außerdem sind die erfindungsgemäßen Gensequenzen zur Erzeugung von verbesserten Mutanten zu verwenden.

In einer nächsten Ausgestaltung bezieht sich die Erfindung auf Plasmide oder Vektoren aufweisend eine oder mehrere der erfindungsgemäßen Nukleinsäuren.

Als Plasmide oder Vektoren kommen im Prinzip alle dem Fachmann für diesen Zweck zur Verfügung stehenden Ausführungsformen in Frage. Derartige Plasmide und Vektoren können Studier et al., Methods Enzymol. 1990, 185, 61-69 oder den Broschüren der Firmen Novagen, Promega, New England Biolabs, Clontech oder Gibco BRL entnommen werden. Weiter bevorzugte Plasmide und Vektoren können gefunden werden in: DNA cloning: a practical approach. Volume I-III, edited by D. M. Glover, IRL Press Ltd., Oxford, Washington DC, 1985, 1987; Denhardt, D. T. and Colasanti, J.: A surey of vectors for regulating expression of cloned DNA in E. coli. In: Rodriguez, R.L. and Denhardt, D. T (eds), Vectors, Butterworth, Stoneham, MA, 1987, pp179-204; Gene expression technology. In: Goeddel, D. V. (eds), Methods in Enzymology, Volume 185, Academic Press, Inc., San Diego, 1990; Sambrook, J., Fritsch, E.F. and Maniatis, T. 1989. Molecular cloning: a laboratory manual, 2nd ed. Cold Spring Harbor Laboratory Press, Cold Spring Harbor, N.Y.
Plasmide, mit denen das die erfindungsgemäße Nukleinsäure aufweisende Genkonstrukt in ganz bevorzugter Weise in den Wirtsorganismus kloniert werden kann, sind: pKK-177-3H (Roche Biochemicals), pBTac (Roche Biochemicals), pKK-233 (Stratagene) oder pET (Novagen). Mit Außnahme der TOPO-Serie, die eine Kanamycin-Resistenz integriert hat, sollten alle anderen Plasmide eine $\beta$-Lactamase für die Ampicilin-Resistenz enthalten.

Gleichfalls ist die Erfindung auf Mikroorganismen aufweisend ein erfindungsgemäßes Plasmid gerichtet.

Der Mikroorganismus, in den die Nukleinsäuren kloniert werden, dient zur Vermehrung und Gewinnung einer ausreichenden Menge des rekombinanten Enzyms. Die Verfahren hierfür sind dem Fachmann wohlbekannt (s. u.). Als Mikroorganismen können im Prinzip alle dem Fachmann für diesen Zweck in Frage kommenden Organismen wie z.B. Prokaryonten oder Eukaryonten, wie Pseudomonas, Streptomyces, Arthrobacter, Bacillus, Staphylococcus, E. coli, Candida, Hansenula, Pichia, Baculoviern herangezogen werden. Vorzugsweise sind E. coli-Stämme für diesen Zweck zu benutzen. Ganz besonders bevorzugt sind: E. coli NM 522, JM109, JM105, RR1, DH5$\alpha$, TOP 10- oder HB101. Plasmide, mit denen das die erfindungsgemäße Nukleinsäure aufweisende Genkonstrukt vorzugsweise in den Wirtsorganismus kloniert wird, sind weiter oben angegeben.

Ein folgender Aspekt der Erfindung richtet sich auf Primer zur Herstellung der erfindungsgemäßen Gensequenzen mittels aller Arten von PCR. Mitumfaßt sind die Sense- und Antisense-Primer codierend für die entsprechenden Aminosäuresequenzen.
Geeignete Primer können prinzipiell nach dem Fachmann bekannten Verfahren gewonnen werden. Das Auffinden der erfindungsgemäßen Primer erfolgt durch Vergleich mit bekannten DNA-Sequenzen oder durch Übersetzung der ins Auge gefaßten Aminosäuresequenzen in das Codon des betrachteten Organismus (z.B. für Streptomyces: Wright et al., Gene 1992, 113, 55-65). Gemeinsamkeiten in der Aminosäuresequenz von Proteinen von sogenannten Superfamilien ist hierfür ebenfalls von Nutzen (Firestine et al., Chemistry & Biology 1996, 3, 779-783). Weitere Informationen diesbezüglich können gefunden werden in Oligonucleotide synthesis: a practical approach, edited by M.J. Gait, IRL Press Ltd, Oxford Washington DC, 1984; PCR Protocols: A guide to methods and applications, edited by M.A. Innis, D.H. Gelfound, J.J. Sninsky and T.J. White. Academic Press, Inc., San Diego, 1990. Äußerst bevorzugt sind folgende Primer:

DIFAAGDSA Seq. 3; AS-Primer

MKVTVVGCT Seq. 4; S-Primer

Die vorliegende Erfindung beschäftigt sich ebenfalls mit der Verwendung der erfindungsgemäßen NADH-Oxidase (NOX) zur Herstellung von chiralen enantiomerenangereicherten organischen Verbindungen, wie z.B. Alkoholen oder Aminosäuren. Die Verwendung erfolgt dabei analog zu den schon bekannten Verfahren (DE10037101, Enzyme Catalysis in Organic Synthesis, Ed.: K. Drauz, H. Waldmann, VCH, 1. Auflage). Weiterhin eigenen sich die erfindungsgemäßen Nukleinsäuren, die für die betrachteten NADH-Oxidase (NOX) codieren, bevorzugt zur Herstellung von Ganzzellkatalysatoren (DE10037115.9 sowie dort zitierte Lit.).

Gegenstand der Erfindung ist ebenfalls ein Verfahren zur Herstellung der NADH-Oxidase aus Lactobacillus, wobei zur vorteilhaften Expression der NADH-Oxidase (NOX) der Mikroorganismus unter aeroben Bedingungen zu kultivieren ist.

Es wurde gefunden, daß Lactobacillus im Standardmedium unter aeroben Bedingungen wesentlich besser wächst als unter den sonst üblichen anaeroben Bedingungen. Bevorzugt ist der Einsatz eines *Lactobacillus brevis,* vorzugsweise DSM 20054. Als aerobe Bedingungen werden erfindungsgemäß vorteilhafterweise solche angesehen, bei denen der Mikroorganismus bei Anzucht in Kolben als Schüttelkultur oder bei Anzucht im Fermenter in Gegenwart meßbarer Konzentrationen von Sauerstoff angezogen wird. Als meßbare Sauerstoffkonzentration wird bezeichnet, daß der Gelöstsauerstoff 1% des Sättigungswertes für Sauerstoff beträgt, wobei der Sauerstoff unter sonst üblichen Bedingungen mit einer Sauerstoffelektrode gemessen wird.

Die erfindungsgemäßen Nukleinsäuren lassen sich also vorzugsweise zur Herstellung von rec-NADH-Oxidase (NOX) einsetzen. Durch rekombinante Techniken, die dem Fachmann hinlänglich bekannt sind (s.u.), gelangt man zu Organismen, welche in der Lage sind, das betrachtete Enzym in für einen technischen Prozeß ausreichender Menge zur Verfügung zu stellen. Die Herstellung der erfindungsgemäßen rec-Enzyme erfolgt nach dem Fachmann bekannten gentechnologischen Verfahren (Sambrook et al. 1989, Molecular cloning: A Laboratory Manual, 2nd Edition, Cold Spring Harbor Laboratory Press, Balbas P & Bolivar F. 1990; Design and construction of expression plasmd vectors in E.coli, Methods Enzymology 185, 14-37; Vectors: A Survey of Molecular Cloning Vectors and Their Uses. R.L. Rodriguez & D.T. Denhardt, Eds: 205-225). Bezüglich der allgemeinen Vorgehensweise (PCR und Fusions-PCR, inverse PCR, Klonierung, Expression etc.) sei auch auf folgende Literatur und das dort zitierte verwiesen: Riley J, Butler R, Finniear R, Jenner D, Powell S, Anand R, Smith JC, Markham AF (1990). A novel, rapid method for the isolation of terminal sequences from yeast artificial chromosome (YAC) clones. Nucl Acids Res. 18, 8186; Triglia T, Peterson MG, Kemp DJ (1988). A procedure for in vitro amplification of DNA segments that lie outside the boundaries of known sequences. Nucleic Acids Res. 16, 8186; Sambrook J, Fritsch EF, Maniatis T (1989). Molecular Cloning. Cold Spring Harbour Laboratory Press; Vectors: A Survey of Molecular Cloning Vectors and Their Uses. R.L. Rodriguez & D.T. Denhardt, II.

Für die Anwendung kann das betrachtete Enzym in freier Form als homogen aufgereinigte Verbindungen oder als rekombinant hergestelltes Enzym verwendet werden. Weiterhin kann das Enzym auch als Bestandteil eines intakten Gastorganismus eingesetzt werden oder in Verbindung mit der aufgeschlossenen und beliebig hoch aufgereinigten Zellmasse des Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialiern - Techniken und Anwendungen", Angew. Chem. 1982, 94, 836-852). Vorteilhafterweise erfolgt die Immobilisierung durch Lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-monocetylether) (Goto et al. Biotechnol. Techniques 1997, 11, 375-378). Die Verwendung als CLECs ist ebenfalls denkbar (St Clair et al. Angew Chem Int Ed Engl 2000 Jan, 39(2), 380-383).

Unter optisch angereicherten (enantiomerenangereicherten, enantiomer angereicherten) Verbindungen wird im Rahmen der Erfindung das Vorliegen einer optischen Antipode im Gemisch mit der anderen in >50 mol-% verstanden.

Der Organismus *Lactobacillus brevis* DSM 20054 oder *Lactobacillus kefir* DSM 20587 ist bei der Deutschen Sammlung für Mirkroorganismen und Zellkulturen unter der entsprechenden Nummer hinterlegt und öffentlich zugänglich.

Unter dem Begriff Nukleinsäuren werden alle Arten von einzelsträngiger oder doppelsträngiger DNA als auch RNA oder Gemische derselben subsumiert.

Unter verbesserten rec-Enzymen werden anspruchsgemäß insbesondere solche verstanden, die ein modifiziertes Substratspektrum aufweisen, aktiver und/oder selektiver oder unter den verwendeten Reaktionsbedingungen stabiler sind.

Auf jeden Fall sind mit dem Begriff Nukleinsäuren, welche für Aminosäuresequenzen codieren, alle Sequenzen umfaßt, die nach Maßgabe der Degeneration des genetischen Codes möglich erscheinen.

Beispiele:

1) Züchtung von *Lactobacillus brevis* zur Gewinnung von NADH-Oxidase

*Lactobacillus brevis* DSM 20054 wurde in einem Standardmedium für Lactobacillen kultiviert. Dabei wurde beobachtet, daß der Stamm unter Sauerstoff-Zufuhr signifikant besser wuchs als unter den für Lactobacillus-Stämmen eigentlich üblichen anaeroben Bedingungen. Ein Enzymtest auf Aktivität einer NADH-Oxidase zeigte, daß der Stamm insbesondere unter diesen ungewöhnlichen Anzuchtbedingungen eine hohe Aktivität einer NADH-Oxidase (NOX) zeigt.
Für die Enzym-Reinigung und -Charakterisierung wurden daher Zellen unter folgenden Bedingungen hergestellt:
Medium: Pro 1 L: 10 g Caseinpepton, tryptisch verdaut; 10 g Fleischextrakt; 5 g Hefeextrakt; 20 g Glucose; 1 g Tween 80; 2 g $K_2HPO_4$; 5 g Na-Acetat; 2 g Diammoniumcitrat; 0,2 g $MgSO_4$ x 7 $H_2O$; 0,05 g $MnSO_4$ x $H_2O$; pH = 6,2-6,5.
Die Anzucht geschieht im Schüttelkolben (mit 2 Schikanen) unter Rühren bei 30oC für 2 Tage. Danach wird die Zellmasse durch Zentrifugation gewonnen, sie kann bei -20oC für längere Zeit gelagert werden.

2) Enzymisolierung

a) Rohextrakt-Gewinnung

Die Zellen wurden durch Naßvermahlung mit Glasperlen aufgeschlossen. Dazu wurde die Bakterienfeuchtmasse (10 g) in 0,1 M Natriumacetat-Puffer, pH 4,5 suspendiert (30 ml) und durch Vermahlen in einer Rührzelle Glasperlenmühle (SCP-Desintegrator, Innomed-Konsult AB, Schweden) bei 4000 rpm, 20 min aufgeschlossen. Die aufgeschlossenen Zellen wurde abzentrifugiert und der Überstand als Rohextrakt bezeichnet.
Der verwendete pH-Wert von 4,5 hat einen entscheidenden Einfluß auf die Enzymausbeute im Rohextrakt, man erhält unter diesen Bedingungen ein Rohextrakt-Präparat mit einer spezifischen Aktivität von 17,8 U/mg und einer Ausbeute von 700 Units, während bei Verwendung eines Puffers mit einem pH-Wert von 7,4 die spezifische Aktivität nur 2,6 U/mg und die Ausbeute 580 Units betrug.
Die Enzymaktivität wurde dabei mit folgendem photometrischen Test (340 nm, 30oC Messtemperatur) gemessen: 0,1 mM NADH; 100 mM Kaliumphosphatpuffer, pH 7,5; 1 mM DTT (= Dithiothreitol); 10 µl Enzymlösung pro 1 ml (evtl. verdünnt). 1 Unit NADH-Oxidase-Aktivität oxidiert 1 µMol NADH / min.

b) Chromatographische Aufreinigung des Enzyms

Nach Herstellung des Rohextrakts in Puffer mit einem pH 4,5 wurde der Rohextrakt sofort umgepuffert und auf eine mit 50 mM TEA pH 7,5 + 3 mM DTT + 200 mM NaCl (=Puffer A) equilibrierte Macro Q Säule (Firma Biorad) aufgetragen (100 ml Säulenvolumen, Fluß 4 ml/min, 10 ml Fraktionen). Die Chromatographie wurde mit einem FPLC-Gerät (Pharmacia) durchgeführt (Druck 0.1 MPa, Raumtemperatur). Die Proteine wurden mittels eines NaCl Stufengradienten eluiert bis 1 M NaCl (Puffer B: Puffer A + 1 M NaCl), wobei folgende Stufen verwendet wurden: 0-2 Säulenvolumina: 0 % B (=200 mM NaCl), 2-5 Säulenvolumina: 40 % B, 5-5.5 Säulenvolumina: 100 % B. In den Fraktionen wurden mit dem photometrischen Test die Enzymaktivität und der Proteingehalt der Fraktionen bestimmt. Die aktiven Fraktionen wurden vereinigt, und bei gleichzeitigem Umpuffern mit Hydroxylapatit-Äquilibrierungspuffer, s.u.) in der Amicon Rührzelle (YM10 Membran, 50 ml) eingeengt.
Dieser Fraktionspool wurde auf eine mit 10 mM Kpi-Puffer pH 6.7 + 3 mM DTT + 200 mM NaCl (=Puffer A) equilibrierte Hydroxylapatit Ceramic Type 20 µm Säule (Firma Biorad) aufgetragen (15 ml Säulenvolumen, Fluß 2 ml/min, 5 ml Fraktionen; FPLC-Gerät, Druck 0.1 MPa, Raumtemperatur). Die Proteine wurden mit einem 3-Stufengradienten bis 500 mM KPi eluiert (Puffer B: Puffer A + 500 mM Kpi) mit folgenden Stufen: 0-3 Säulenvolumina 0% B, 3-7 Säulenvolumina 30 % B, 7-9 Säulenvolumina 50 % B, danach ansteigend bis 100 % B in 3 Säulenvolumina. Die aktiven Fraktionen vereinigt und wie oben aufkonzentriert und mit dem für die Phenylsepharose benötigten Puffer umgepuffert.
Dieses Präparat wurde nach Zugabe von Ammoniumsulfat (1,2 M Endkonzentration in der Probe) auf eine mit 50 mM TEA pH 7,5 + 3 mM DTT + 1,2 M (NH4)2SO4 (=Puffer A) equilibrierte Phenylsepharose high sub (Firma Pharmacia) aufgetragen (25 ml Säulenvolumen, Fluß 1 ml/min, Fraktionen 3 ml; FPLC-Gerät, Druck 0,1 Mpa, Raumtemperatur). Die Proteine wurden mit einem 3-Stufengradienten bis 0 M Ammoniumsulfat eluiert (Puffer B: Puffer A ohne Ammoniumsulfat) mit folgenden Stufen: 1 Säulenvolumen 0 % B, 1 Säulenvolumen 20 % B, 1 Säulenvolumen 60 % B, 2 Säulenvolumina 100 % B). Die aktiven Fraktionen wurden vereinigt, mit einer Amicon-Ultrafiltrationszelle eingeengt, und erneut mit Ammoniumsulfat versetzt.

Die letzte Reinigungsstufe, die mit der aufkonzentrierten Fraktion der Phenylsepharose high sub durchgeführt wurde, ist eine Feinreinigung durch Chromatographie an Phenylsepharose 650C-Material (Firma Tosohaas), equilibriert mit 50 mM TEA pH 7,5 + 3 mM DTT + 1,2 M (NH4)2SO4 (=Puffer A). Chromatographiebedingungen: Säulenvolumen 2 ml, Fluß 1 ml/min; FPLC-Gerät, Druck 0,1 MPa, Raumtemperatur, Fraktionen 2 ml). Die Proteine werden mit einen 4-Stufengradienten bis 0 M Ammoniumsulfat eluiert (Puffer B: Puffer A ohne Ammoniumsulfat) mit folgenden Stufen: 10 Säulenvolumina 0% B, 5 Säulenvolumina 20 % B, 5 Säulenvolumina 50 % B, 5 Säulenvolumina 70 % B, 10 Säulenvolumina 100 % B).

Die Ausbeuten der verschiedenen Chromatographieschritte an Enzymaktivität und Protein sind in Tabelle 1 zusammengefaßt.

Tabelle 1: Reinigung der NADH-Oxidase aus *Lactobacillus brevis* DSM 20054 (OH-Apatit = Hydroxyapatit; PS = Phenylsepharose)

| Probe | Aktivität [U/ml] | Spez. Akt. [U/mg] | Gesamt-Aktivität [U] | Ausbeute [%] | Anreich.-Faktor |
|---|---|---|---|---|---|
| Rohextrakt | 35,1 | 17,8 | 702 | 100 | 1 |
| Macro Q | 77,3 | 10,4 | 270 | 38 | 0,6 |
| OH-Apatit | 32,2 | 12,2 | 103 | 15 | 0,7 |
| PS | 44 | 58,6 | 87 | 12 | 3,3 |
| PS 650C | 98 | 116 | 98 | 14 | 6,5 |

Eine SDS-PAGE der entsprechend Tabelle 1 gereinigten Fraktionen zeigt, daß die Enzymfraktion nach dem letzten Schritt nur noch 2 Proteine enthält, deren Untereinheiten Molmassen bei 38 und 50 kDa zeigen. Man kann diese beiden Proteinbanden nach Blotting einzeln N-terminal ansequenzieren, dabei zeigt ein Vergleich mit in Datenbanken zugänglichen Sequenzen, daß es bei der 38 kDa-Bande sehr hohe Ähnlichkeiten mit bekannten Lactat-Dehydrogenasen und bei der 50 kDa-Bande mit NADH-Oxidasen gibt.

3) Biochemische Kenndaten der NADH-Oxidase aus *L. brevis*

Die biochemischen Daten wurden mit einem durch 4 Chromatographie-Schritte gereinigten Enzympräparat bestimmt.

a) pH-Abhängigkeit der Oxidation

Das pH-Optimum wurde im Bereich pH 4,0 bis 10,0 gemessen. Je nach pH-Wert wurden unterschiedliche Puffer verwendet. Als Kontrolle des Puffereinflusses wurden bei Pufferwechsel dieser pH-Wert doppelt in den verschiedenen Puffern gemessen (pH 7,0 und pH 8,5). Verwendet wurde für den Bereich pH 4,0-7,0 0,1 M Citronensäure/Na2PO4, für den Bereich pH 7,0-8,5 0,1 M TEA und für den Bereich pH 8,5-10,0 0,1 M Glycin/NaOH. Figur 1 zeigt, daß das Optimum der NADH-Oxidation bei 8,0 bis 8,5 liegt.

b) pH-Stabilität der NADH-Oxidase

Zur Bestimmung der pH-Stabilität wurde die NADH-Oxidase für 8 h in den unter 3a) aufgeführten Puffern inkubiert, dann wurde im Standardtest die Restaktivität bestimmt. Fig. 2 zeigt, daß das Enzym ein erstes scharfes Stabilitätsoptimum bei pH 5,0 hat, im Bereich von 5,5 - 7,5 mäßig stabil ist und ein zweites Optimum bei pH 8,5 zeigt. Bei 8,5 ist das Enzym sowohl im TEA/NaOH- als auch im Glycin/NaOH-Puffer nach 8 h noch zu 100% stabil. Die Figur verdeutlicht, daß das Enzym im leicht alkalischen Bereich (pH 8 - 9) in einem breiteren Bereich stabil ist. Dieser Bereich ist für die Anwendung interessant, wenn die NADH-Oxidase für die Oxidation mit einer NAD-abhängigen Dehydrogenase gekoppelt werden soll, da das pH-Optimum vieler Dehydrogenasen für die Oxidation im leicht alkalischen liegt.

c) Temperaturoptimum der NADH-Oxidase

Aktivitätstests bei verschiedenen Temperaturen zeigen, daß die NADH-Oxidase maximale Aktivität bei 40oC zeigt (Figur 3).

d) Temperaturstabilität der NADH-Oxidase

Zur Bestimmung der Temperaturstabilität wurden Enzymproben bei 30oC und 42oC gelagert, zu verschiedenen Zeiten Proben entnommen und die Restaktivität bestimmt. Die Auswertung zeigt, daß das Enzym bei 30oC für ca. 400 min praktisch stabil ist, danach scheint es mit einer langsamen Kinetik zu desaktivieren (Halbwertszeit ca. 18 h). Bei 42oC desaktiviert das Enzym anfangs (bis ca. 120 min) relativ rasch, danach scheint es bei ca. 50% Restaktivität über längere Zeit (mind. 16 h) stabil.

e) Bestimmung des KM-Werts für NADH für die NADH-Oxidase

Für das Substrat NADH wurde die Konzentration im Bereich von 0,0013 bis 0,52 mM variiert und photometrisch die Aktivität bestimmt. Die Auswertung zeigt, daß bei 0,1 mM maximale Aktivität erreicht ist. Höhere NADH-Konzentrationen (bis 0,5 mM) zeigen nur eine schwache Überschuß-Inhibierung. Der Km-Wert errechnet sich zu 24 μM. Diese hohe Affinität des Enzyms für NADH ist sehr vorteilhaft für Anwendungen, bei denen NAD+ durch Oxidation von NADH regeneriert werden soll, da diese eine möglichst vollständige Oxidation von NADH voraussetzen.

4) Proteinchemische Kenndaten der NADH-Oxidase aus *Lactobacillus brevis*

a) N-terminale Aminosäure-Sequenz

Als Ergebnis mehrerer Anreicherungen ergab sich immer wieder ein Protein, dessen Untereinheiten im SDS-PAGE eine Molmasse von 50 kDa aufweist. Für dieses Protein konnten die ersten 27 Aminosäuren des N-Terminus bestimmt werden (automatisierter Edman-Abbau mit einem Automated Sequencer 4774 (Applied Biosystems) mit online HPLC 120 A), die Sequenz lautet: MKVTVVGCTHAGTFAIKQILAEEPDADXXVY (Seq 5). In der Sequenz ist ein konserviertes Motiv unterstrichen, das bei NADH-Oxidasen als FAD-Bindungsmotiv fungiert, und eine leichte Abwandlung der bekannten ADP-Bindungsstelle GXGXXG ist. Datenbank-Recherchen (BLAST) zeigen bereits für dieses Protein-Teilstück Übereinstimmung mit bekannten NADH-Oxidasen.

b) Bestimmung der Protein- und Gensequenz

Die Ergebnisse der Datenbank-Recherche zeigen in einigen Teilbereichen Sequenzen auf, die in nahezu allen NADH-Oxidasen oder in verwandten NADH-Peroxidasen vorkommen. Als ein solcher konservierter Bereich wird die Aminosäure-Sequenz DIFAAGDSA (Seq. 3) angesehen. Für diese Sequenz wird ein Antisense-Codon als "C-terminaler" Primer eingesetzt, und mit einem Primer, der für den N-Terminus MKVTVVGCT (Seq. 4) codiert in einer PCR eingesetzt. Die PCR ergibt ein 777 bp großes Stück, das 59% Identität zur NADH-Oxidase (NOX) aus Enterococcus aufweist. Durch Verdau genomischer DNA mit 4 verschiedenen Restriktions-Endonucleasen (EcoRV, Pvu II, Sca I und Stu I) wird eine Genbank angelegt. Mit spezifischen Primern, abgeleitet aus den Schnittstellen der Restristionsenzyme und Sequenzen aus dem 777 bp-Teilstück, werden dann mittels PCR weitere Sequenzstücke erhalten, wobei mit Hilfe überlappender DNA-Sequenzen und dem Stop-Codon die Gesamtsequenz definiert werden kann.

Die Sequenz für die NADH-Oxidase (NOX) aus L. brevis ist im oben aufgeführten Sequenzprotokoll (Seq. 1) wiedergegeben.

5) Verwendung der NADH-Oxidase aus *Lactobacillus brevis* für die Coenzym-Regenerierung

Eine typische Anwendung für die NADH-Oxidase stellt die Regenerierung von NAD+ dar. Dies kann beispielsweise erforderlich sein, wenn NAD-abhängige Dehydrogenasen für die Oxidation eingesetzt werden, wie es in allgemeiner Weise vorab gezeigt wurde (Schema 1). Im folgenden Beispiel ist dies für die Darstellung eines enantiomerenreinen Alkohols entsprechend der folgenden Gleichungen nachgewiesen worden:

$$\text{(R,S)-Alkohol} + \text{NAD}^+ \longrightarrow \text{S-Alkohol} + \text{Keton} + \text{NADH} + \text{H}^+ \quad (4)$$

$$\text{NADH} + \text{H}^+ + \tfrac{1}{2}\,\text{O}_2 \longrightarrow \text{NAD}^+ + \text{H}_2\text{O} \quad (5)$$

mit einer R-spezifischen Alkohol-Dehydrogenase als Katalysator für Reaktion (4) und der NADH-Oxidase für (5).

Als R-spezifische Alkohol-Dehydrogenase wurde in diesem Beispiel eine NAD-Mutante der R-Alkohol-Dehydrogenase aus *Lactobacillus brevis* (recADHG37D (NAD-Mutante) DE10037101.9) eingesetzt, gekoppelt mit der gereinigten NADH-Oxidase aus *Lactobacillus brevis.* Als Alkohol wurde (R,S)-Phenylethanol eingesetzt, die Reaktion wurde als Batch-Umsetzung durchgeführt. Eingesetzt wurden: 50 mM (R,S)-Phenylethanol, 2 mM NADH, 50 mM TEA-Puffer pH 7,0 mit 1mM $MgCl_2$ und mit 5 mM DTT, 1 U recADHG37D und 2 U NADH-Oxidase.

Proben wurden nach 0, 1, 2, 3, 4, 6, 9 und 24 h entnommen und gaschromatographisch aufgetrennt (Säule: CP-Chirasil-DEX CB Länge: 25 m, Durchmesser: 25 μm (Fa. Chrompack). Temperaturprogramm: 5 min bei 60oC, dann 5oC/min bis auf 190oC; Säulenfluß 1,3 ml/min; Gas: Helium. Dabei wurde sowohl der Acetophenonpeak (Produkt; Retentionszeit = 16,9 min), als auch die Enantiomeren des Phenylethanol (Edukte; Retentionszeiten R-Phenylethanol = 20,8 min und S-Phenylethanol = 21,1 min) aufgezeigt. Figur 4 zeigt das GC-Spektrum einer Probe, in der der R-Alkohol nahezu komplett oxidiert worden ist, dafür ist eine entsprechende Menge des Oxidationsprodukts Acetophenon zu sehen. Als Kontrolle wurde der gleiche Ansatz, nur ohne NADH-Oxidase-Zugabe durchgeführt. Er zeigt in der gaschromatographischen Analyse keine signifikante Änderung der beiden Alkohole, lediglich eine kleine Menge an Acetophenon ist nachweisbar, die möglicherweise auf eine Spontanoxidation von NADH durch Luftsauerstoff zurückzuführen ist.

## Patentansprüche

1. Nukleinsäure codierend für eine NADH-Oxidase gemäß SEQ ID NO:1 oder die zu dieser Sequenz komplementäre Sequenz.

2. NADH-Oxidase codiert durch eine Nukleinsäure gemäß Anspruch 1.

3. NADH-Oxidase gemäß SEQ ID NO:2.

4. Plasmide, Vektoren aufweisend eine oder mehrere klonierte Nukleinsäuren nach Anspruch 1.

5. Primer zur Herstellung der Nukleinsäuren nach Anspruch 1.

6. Verwendung der NADH-Oxidasen (NOX) gemäß Anspruch 2 oder 3 zur Herstellung von chiralen enantiomerenangereicherten organischen Verbindungen, wie z.B. Alkoholen oder Aminosäuren.

7. Verwendung der Nukleinsäuren gemäß Anspruch 1 zur Herstellung von Ganzzellkatalysatoren.

8. Mikroorganismus aufweisend ein Plasmid nach Anspruch 4.

9. Verfahren zur Herstellung von NADH-Oxidasen aus Lactobacillus gemäß Anspruch 2 oder 3,
**dadurch gekennzeichnet, dass**
man die Mikroorganismen gemäß Anspruch 8 unter aeroben Bedingungen kultiviert.

## Claims

1. Nucleic acid coding for an NADH oxidase according to SEQ ID NO:1 or the sequence complementary thereto.

2. NADH oxidase encoded by a nucleic acid according to Claim 1.

3. NADH oxidase according to SEQ ID NO:2.

4. Plasmids, vectors comprising one or more cloned nucleic acids according to Claim 1.

5. Primer for producing the nucleic acids according to Claim 1.

6. Use of the NADH oxidases (NOX) according to Claim 2 or 3 for producing chiral enantiomer-enriched organic compounds, for example alcohols or amino acids.

7. Use of the nucleic acids according to Claim 1 for producing whole-cell catalysts.

**8.** Microorganism comprising a plasmid according to Claim 4.

**9.** Process for producing NADH oxidases from Lactobacillus according to Claim 2 or 3, **characterized in that** the microorganisms according to Claim 8 are cultivated under aerobic conditions.

## Revendications

**1.** Acide nucléique codant pour une NADH-oxydase selon SEQ ID NO:1 ou la séquence complémentaire à cette séquence.

**2.** NADH-oxydase codée par un acide nucléique selon la revendication 1.

**3.** NADH-oxydase selon SEQ ID NO:2.

**4.** Plasmides, vecteurs présentant un ou plusieurs acides nucléiques clonés selon la revendication 1.

**5.** Amorceur pour la préparation des acides nucléiques selon la revendication 1.

**6.** Utilisation des NADH-oxydases (NOX) selon la revendication 2 ou 3 pour la préparation de composés organiques chiraux enrichis en énantiomères, comme par exemple des alcools ou des acides aminés.

**7.** Utilisation des acides nucléiques selon la revendication 1 pour la préparation de catalyseurs à cellules entières.

**8.** Micro-organisme présentant un plasmide selon la revendication 4.

**9.** Procédé pour la préparation de NADH-oxydases à partir de Lactobacillus selon la revendication 2 ou 3, **caractérisé en ce que**
les micro-organismes selon la revendication 8 sont cultivés dans des conditions aérobies.

Fig. 1:

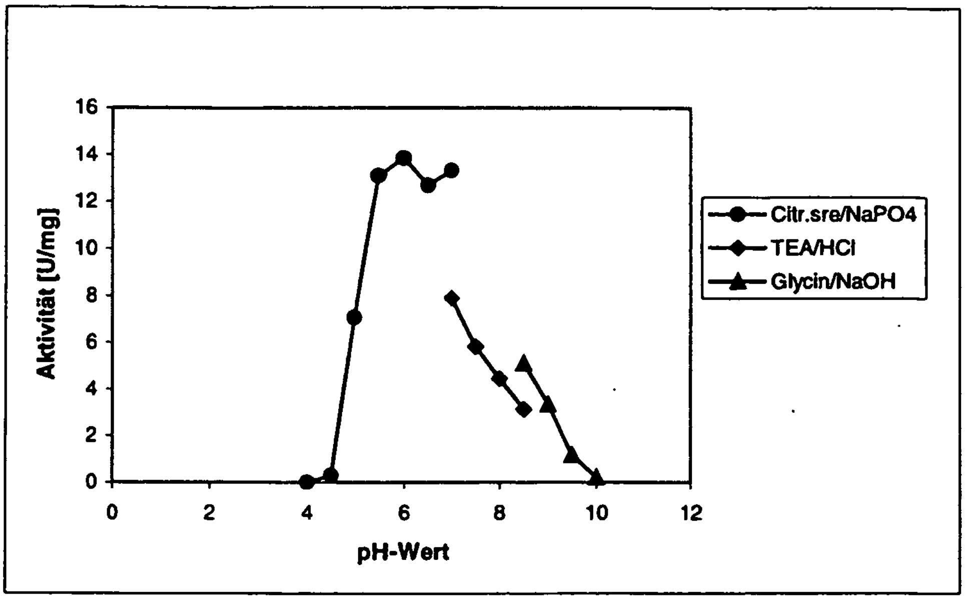
Aktivität [U/mg]
16
14
12
10
8
6
4
2
0
0
2
4
6
8
10
12
pH-Wert
Citr.sre/NaPO4
TEA/HCl
Glycin/NaOH

Fig. 2:

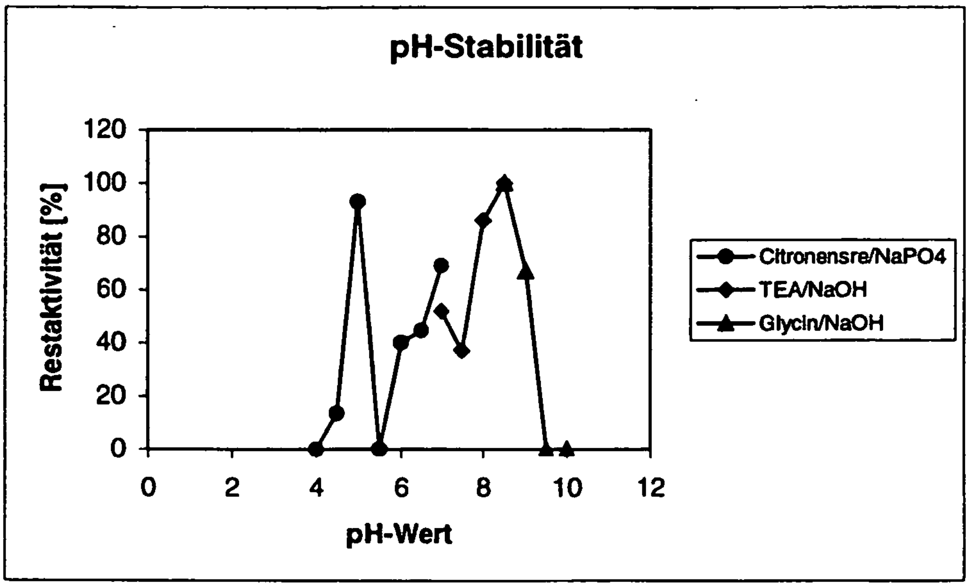
pH-Stabilität
Restaktivität [%]
120
100
80
60
40
20
0
0
2
4
6
8
10
12
pH-Wert
Citronensre/NaPO4
TEA/NaOH
Glycin/NaOH

Fig. 3:

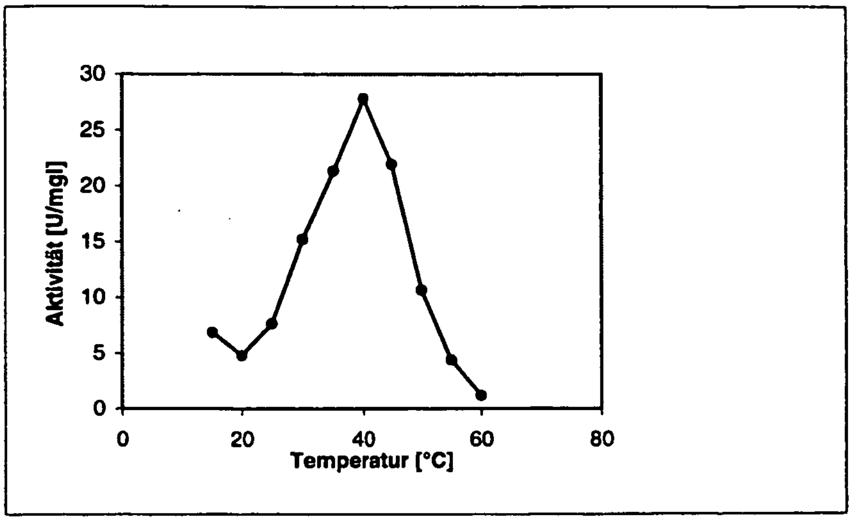
Aktivität [U/mgl]
Temperatur [°C]
0
5
10
15
20
25
30
0
20
40
60
80

Fig. 4:

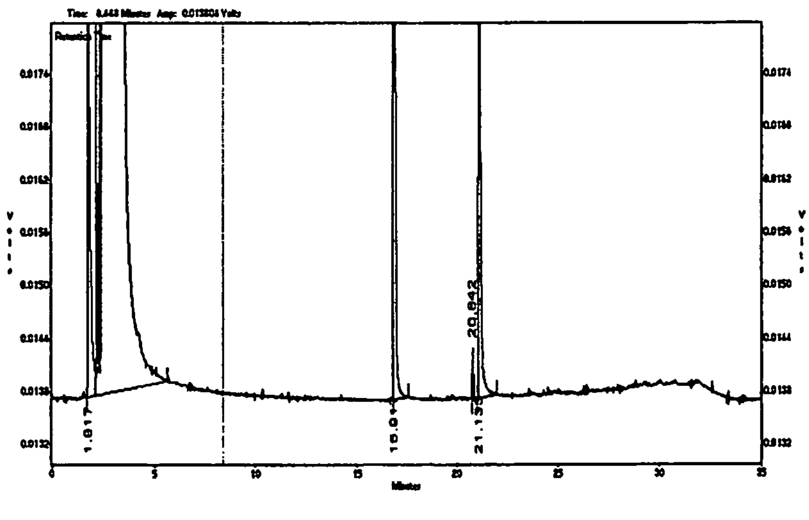
1.617
16.0
20.842
21.13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10037101
- DE 10037115


### In der Beschreibung aufgeführte Nicht-Patentliteratur


- Enzyme Catalysis in Organic Synthesis. vol. 1, 721
- **LOPEZ DE FELIPE, F et al.** *J. Bacteriol.,* 1998, vol. 180, 3804-08
- **NISHIYAMA, Y. et al.** *J. Bacteriol.,* 2001, vol. 183, 2431-2438
- **SCHMIDT, H.L. et al.** *Eur. J. Biochem.,* 1986, vol. 156, 149-55
- **KOIKE, K. J. et al.** *Biochem.,* 1985, vol. 97, 1279-88
- **HIGUCHI, M. et al.** *Biosci. Biotechnol. Biochem.,* 1994, vol. 58, 1603-07
- **KLOMKES, M. ; ALTDORF, R. ; OHLENBUSCH, H. D.** *Biol. Chem.,* 1985, vol. 366, 963-9
- **ARCARI, P. et al.** *J. Biol. Chem.,* 2000, vol. 275, 895-900
- **ERDMANN, H. et al.** *J. Mol. Biol.,* 1993, vol. 230, 1086-8
- **COCCO, D. et al.** *Eur. J. Biochem.,* 1988, vol. 174, 267-71
- **STUDIER et al.** *Methods Enzymol.,* 1990, vol. 185, 61-69
- DNA cloning: a practical approach. IRL Press Ltd, 1985, vol. I-III
- **DENHARDT, D. T. ; COLASANTI, J.** A surey of vectors for regulating expression of cloned DNA in E. coli. Butterworth, 1987, 179-204
- Gene expression technology. Methods in Enzymology. Academic Press, Inc, 1990, vol. 185
- **SAMBROOK, J. ; FRITSCH, E.F. ; MANIATIS, T.** Molecular cloning: a laboratory manual. Cold Spring Harbor Laboratory Press, 1989
- **WRIGHT et al.** *Gene,* 1992, vol. 113, 55-65
- **FIRESTINE et al.** *Chemistry & Biology,* 1996, vol. 3, 779-783
- Oligonucleotide synthesis: a practical approach. IRL Press Ltd, 1984
- PCR Protocols: A guide to methods and applications. Academic Press, Inc, 1990
- Enzyme Catalysis in Organic Synthesis. vol. 1
- **SAMBROOK et al.** Molecular cloning: A Laboratory Manual. Cold Spring Harbor Laboratory Press, 1989
- **BALBAS P ; BOLIVAR F.** Design and construction of expression plasmd vectors in E.coli. *Methods Enzymology,* 1990, vol. 185, 14-37
- Vectors: A Survey of Molecular Cloning Vectors and Their Uses. 205-225
- **RILEY J ; BUTLER R ; FINNIEAR R ; JENNER D ; POWELL S ; ANAND R ; SMITH JC ; MARKHAM AF.** A novel, rapid method for the isolation of terminal sequences from yeast artificial chromosome (YAC) clones. *Nucl Acids Res.,* 1990, vol. 18, 8186
- **TRIGLIA T ; PETERSON MG ; KEMP DJ.** A procedure for in vitro amplification of DNA segments that lie outside the boundaries of known sequences. *Nucleic Acids Res.,* 1988, vol. 16, 8186
- **SAMBROOK J ; FRITSCH EF ; MANIATIS T.** Molecular Cloning. Cold Spring Harbour Laboratory Press, 1989
- Vectors: A Survey of Molecular Cloning Vectors and Their Uses. vol. II
- Immobilisierte Biomaterialiern - Techniken und Anwendungen. **BHAVENDER P. SHARMA ; LORRAINE F. BAILEY ; RALPH A. MESSING.** Angew. Chem. 1982, vol. 94, 836-852
- **DORDICK et al.** *J. Am. Chem. Soc.,* vol. 194 (116), 5009-5010
- **OKAHATA et al.** *Tetrahedron Lett.,* 1997, vol. 38, 1971-1974
- **ADLERCREUTZ et al.** *Biocatalysis,* 1992, vol. 6, 291-305
- **GOTO et al.** *Biotechnol. Techniques,* 1997, vol. 11, 375-378
- **ST CLAIR et al.** *Angew Chem Int Ed Engl,* 03. Januar 2000, vol. 9 (2), 380-383

SEQUENZPROTOKOLL

<110> Degussa AG

<120> NADH-Oxidase aus Lactobacillus

<130> 010281 AM

<140>
<141>

<160> 5

<170> PatentIn Ver. 2.1

<210> 1
<211> 1353
<212> DNA
<213> Lactobacillus brevis

<220>
<221> CDS
<222> (1)..(1353)

<400> 1

```
atg aaa gtc aca gtt gtt ggt tgt aca cat gcc gga acc ttt gcg att    48
Met Lys Val Thr Val Val Gly Cys Thr His Ala Gly Thr Phe Ala Ile
 1               5                  10                  15

aaa caa atc ttg gcc gaa cac cct gat gcc gaa gtg acc gtc tac gaa    96
Lys Gln Ile Leu Ala Glu His Pro Asp Ala Glu Val Thr Val Tyr Glu
            20                  25                  30

cgt aac gat gtc att tca ttt ctc tct tgt gga atc gcc ctt tac ctg    144
Arg Asn Asp Val Ile Ser Phe Leu Ser Cys Gly Ile Ala Leu Tyr Leu
        35                  40                  45

ggg gga aaa gtc gct gat ccg caa ggc ctc ttt tat tca agt cct gaa    192
Gly Gly Lys Val Ala Asp Pro Gln Gly Leu Phe Tyr Ser Ser Pro Glu
    50                  55                  60

gaa ctc caa aaa tta ggc gct aat gtc caa atg aat cac aat gtt tta    240
Glu Leu Gln Lys Leu Gly Ala Asn Val Gln Met Asn His Asn Val Leu
65                  70                  75                  80

gcg atc gat cct gat caa aag aca gtg acc gtt gag gac tta acc agt    288
Ala Ile Asp Pro Asp Gln Lys Thr Val Thr Val Glu Asp Leu Thr Ser
                85                  90                  95

cat gca caa acg act gag tca tac gat aaa cta gtc atg acg tct ggt    336
His Ala Gln Thr Thr Glu Ser Tyr Asp Lys Leu Val Met Thr Ser Gly
            100                 105                 110

tct tgg cca att gtc ccc aag att ccg ggc atc gat agc gat cgc gtt    384
Ser Trp Pro Ile Val Pro Lys Ile Pro Gly Ile Asp Ser Asp Arg Val
        115                 120                 125

aag ctc tgc aaa aac tgg gca cat gcg caa gct cta atc gaa gat gct    432
Lys Leu Cys Lys Asn Trp Ala His Ala Gln Ala Leu Ile Glu Asp Ala
```

```
        130                 135                 140

aag gaa gcc aag cgg att acc gtt att ggt gcc ggc tat att ggt gct    480
Lys Glu Ala Lys Arg Ile Thr Val Ile Gly Ala Gly Tyr Ile Gly Ala
145                 150                 155                 160

gaa cta gca gaa gcc tac tcc act act ggt cat gac gta acc tta att    528
Glu Leu Ala Glu Ala Tyr Ser Thr Thr Gly His Asp Val Thr Leu Ile
                165                 170                 175

gat gcg atg gac cgg gtt atg ccc aag tac ttt gat gct gat ttt acg    576
Asp Ala Met Asp Arg Val Met Pro Lys Tyr Phe Asp Ala Asp Phe Thr
            180                 185                 190

gat gtc att gaa caa gat tat cgg gat cac ggt gtc caa ctt gcc tta    624
Asp Val Ile Glu Gln Asp Tyr Arg Asp His Gly Val Gln Leu Ala Leu
        195                 200                 205

agt gaa acg gtt gaa agc ttt act gat agt gca act ggg ttg acc att    672
Ser Glu Thr Val Glu Ser Phe Thr Asp Ser Ala Thr Gly Leu Thr Ile
    210                 215                 220

aag act gat aag aac agc tat gaa acg gat ctc gct att tta tgc att    720
Lys Thr Asp Lys Asn Ser Tyr Glu Thr Asp Leu Ala Ile Leu Cys Ile
225                 230                 235                 240

ggc ttt aga cca aat acc gac ctg ctg aaa ggc aaa gtc gat atg gca    768
Gly Phe Arg Pro Asn Thr Asp Leu Leu Lys Gly Lys Val Asp Met Ala
                245                 250                 255

cca aac ggc gcg att att acg gat gac tac atg cgt tct tct aac cct    816
Pro Asn Gly Ala Ile Ile Thr Asp Asp Tyr Met Arg Ser Ser Asn Pro
            260                 265                 270

gat att ttc gcc gct ggt gac agt gct gct gtg cac tac aac cca acc    864
Asp Ile Phe Ala Ala Gly Asp Ser Ala Ala Val His Tyr Asn Pro Thr
        275                 280                 285

cat cag aat gct tac att ccc tta gca act aac gcg gtg cgt caa ggt    912
His Gln Asn Ala Tyr Ile Pro Leu Ala Thr Asn Ala Val Arg Gln Gly
    290                 295                 300

atc cta gtc ggt aaa aac cta gtt aag ccg acc gtc aag tat atg gga    960
Ile Leu Val Gly Lys Asn Leu Val Lys Pro Thr Val Lys Tyr Met Gly
305                 310                 315                 320

aca caa tca tct tct ggt ttg gca ctc tat gat cgg acg atc gtc tca   1008
Thr Gln Ser Ser Ser Gly Leu Ala Leu Tyr Asp Arg Thr Ile Val Ser
                325                 330                 335

act ggt tta acg cta gca gct gca aaa caa caa ggg gtg aac gct gaa   1056
Thr Gly Leu Thr Leu Ala Ala Ala Lys Gln Gln Gly Val Asn Ala Glu
            340                 345                 350

caa gtg att gtt gaa gat aat tat cgc cca gag ttt atg ccg tca act   1104
Gln Val Ile Val Glu Asp Asn Tyr Arg Pro Glu Phe Met Pro Ser Thr
        355                 360                 365

gaa ccc gtt ttg atg tca tta gtc ttt gac ccc gac aca cac cgg atc   1152
```

```
Glu Pro Val Leu Met Ser Leu Val Phe Asp Pro Asp Thr His Arg Ile
    370                 375                 380

tta ggt ggt gcg tta atg agt aaa tac gat gtt tca caa tcg gcc aac      1200
Leu Gly Gly Ala Leu Met Ser Lys Tyr Asp Val Ser Gln Ser Ala Asn
385                 390                 395                 400

acc ctt tct gtt tgc atc caa aac gaa aat aca att gat gac tta gcg      1248
Thr Leu Ser Val Cys Ile Gln Asn Glu Asn Thr Ile Asp Asp Leu Ala
                405                 410                 415

atg gtt gat atg ctc ttc cag cct aac ttt gat cga cca ttc aac tac      1296
Met Val Asp Met Leu Phe Gln Pro Asn Phe Asp Arg Pro Phe Asn Tyr
            420                 425                 430

cta aac atc tta gcg caa gct gct cag gca aag gtt gcc caa tca gtt      1344
Leu Asn Ile Leu Ala Gln Ala Ala Gln Ala Lys Val Ala Gln Ser Val
        435                 440                 445

aac gct taa                                                          1353
Asn Ala
    450
```

<210> 2
<211> 450
<212> PRT
<213> Lactobacillus brevis

<400> 2

```
Met Lys Val Thr Val Val Gly Cys Thr His Ala Gly Thr Phe Ala Ile
1               5                   10                  15
Lys Gln Ile Leu Ala Glu His Pro Asp Ala Glu Val Thr Val Tyr Glu
            20                  25                  30
Arg Asn Asp Val Ile Ser Phe Leu Ser Cys Gly Ile Ala Leu Tyr Leu
        35                  40                  45
Gly Gly Lys Val Ala Asp Pro Gln Gly Leu Phe Tyr Ser Ser Pro Glu
    50                  55                  60
Glu Leu Gln Lys Leu Gly Ala Asn Val Gln Met Asn His Asn Val Leu
65                  70                  75                  80
Ala Ile Asp Pro Asp Gln Lys Thr Val Thr Val Glu Asp Leu Thr Ser
                85                  90                  95
His Ala Gln Thr Thr Glu Ser Tyr Asp Lys Leu Val Met Thr Ser Gly
            100                 105                 110
Ser Trp Pro Ile Val Pro Lys Ile Pro Gly Ile Asp Ser Asp Arg Val
        115                 120                 125
Lys Leu Cys Lys Asn Trp Ala His Ala Gln Ala Leu Ile Glu Asp Ala
    130                 135                 140
Lys Glu Ala Lys Arg Ile Thr Val Ile Gly Ala Gly Tyr Ile Gly Ala
145                 150                 155                 160
Glu Leu Ala Glu Ala Tyr Ser Thr Thr Gly His Asp Val Thr Leu Ile
                165                 170                 175
Asp Ala Met Asp Arg Val Met Pro Lys Tyr Phe Asp Ala Asp Phe Thr
            180                 185                 190
Asp Val Ile Glu Gln Asp Tyr Arg Asp His Gly Val Gln Leu Ala Leu
        195                 200                 205
Ser Glu Thr Val Glu Ser Phe Thr Asp Ser Ala Thr Gly Leu Thr Ile
    210                 215                 220
Lys Thr Asp Lys Asn Ser Tyr Glu Thr Asp Leu Ala Ile Leu Cys Ile
```

```
225                 230                 235                 240
Gly Phe Arg Pro Asn Thr Asp Leu Leu Lys Gly Lys Val Asp Met Ala
                245                 250                 255
Pro Asn Gly Ala Ile Ile Thr Asp Asp Tyr Met Arg Ser Ser Asn Pro
            260                 265                 270
Asp Ile Phe Ala Ala Gly Asp Ser Ala Ala Val His Tyr Asn Pro Thr
        275                 280                 285
His Gln Asn Ala Tyr Ile Pro Leu Ala Thr Asn Ala Val Arg Gln Gly
    290                 295                 300
Ile Leu Val Gly Lys Asn Leu Val Lys Pro Thr Val Lys Tyr Met Gly
305                 310                 315                 320
Thr Gln Ser Ser Ser Gly Leu Ala Leu Tyr Asp Arg Thr Ile Val Ser
                325                 330                 335
Thr Gly Leu Thr Leu Ala Ala Ala Lys Gln Gln Gly Val Asn Ala Glu
            340                 345                 350
Gln Val Ile Val Glu Asp Asn Tyr Arg Pro Glu Phe Met Pro Ser Thr
        355                 360                 365
Glu Pro Val Leu Met Ser Leu Val Phe Asp Pro Asp Thr His Arg Ile
    370                 375                 380
Leu Gly Gly Ala Leu Met Ser Lys Tyr Asp Val Ser Gln Ser Ala Asn
385                 390                 395                 400
Thr Leu Ser Val Cys Ile Gln Asn Glu Asn Thr Ile Asp Asp Leu Ala
                405                 410                 415
Met Val Asp Met Leu Phe Gln Pro Asn Phe Asp Arg Pro Phe Asn Tyr
            420                 425                 430
Leu Asn Ile Leu Ala Gln Ala Ala Gln Ala Lys Val Ala Gln Ser Val
        435                 440                 445
Asn Ala
    450
```

<210> 3
<211> 9
<212> PRT
<213> Künstliche Sequenz

<220>
<223> Beschreibung der künstlichen Sequenz:AS_Primer

<400> 3

```
Asp Ile Phe Ala Ala Gly Asp Ser Ala
1               5
```

<210> 4
<211> 9
<212> PRT
<213> Künstliche Sequenz

<220>
<223> Beschreibung der künstlichen Sequenz:S_Primer

<400> 4

```
Met Lys Val Thr Val Val Gly Cys Thr
1               5
```

<210> 5
<211> 31
<212> PRT
<213> Lactobacillus brevis

<400> 5

```
Met Lys Val Thr Val Val Gly Cys Thr His Ala Gly Thr Phe Ala Ile
 1               5                  10                  15

Lys Gln Ile Leu Ala Glu Glu Pro Asp Ala Asp Xaa Xaa Val Tyr
            20                  25                  30
```